# EUROPÄISCHE PATENTSCHRIFT

(11) **EP 2 641 619 B1**
(45) Veröffentlichungstag und Bekanntmachung des Hinweises auf die Patenterteilung: **01.10.2014**
(21) Anmeldenummer: 12401044.8
(22) Anmeldetag: 19.03.2012
(51) Int. Cl.: A61L 2/18, A61L 2/24, A61B 19/00, D06F 39/00

(54) **Reinigungs- und Desinfektionsautomat**
Cleaning and disinfection machine
Automate de nettoyage et de désinfection

(43) Veröffentlichungstag der Anmeldung: 25.09.2013
(73) Patentinhaber: Miele & Cie. KG, 33332 Gütersloh (DE)
(72) Erfinder: Dyck, Heinrich, 33719 Bielefeld (DE); Leifeld, Ludger, 59227 Ahlen (DE)

(56) Entgegenhaltungen:
- EP-A1- 1 902 670
- DE-A1- 2 556 035
- JP-A- 1 126 941
- JP-A- 2007 260 034

## Beschreibung

Die Erfindung betrifft einen Reinigungs- und Desinfektionsautomat insbesondere für medizinische Instrumente und Geräte, mit einem Spülbottich, der einen mit einer Tür fluiddicht verschließbaren Spülraum bereitstellt, mit einer im Spülraum angeordneten Fluidabgabeeinrichtung, die der Abgabe von Spülflotte einerseits und von Trocknungsluft andererseits dient.

Reinigungs- und Desinfektionsautomaten der vorbeschriebenen Art sind aus dem Stand der Technik an sich bekannt. Sie verfügen über einen Spülbottich, der einen Spülraum bereitstellt. Im bestimmungsgemäßen Verwendungsfall dient der Spülraum der Aufnahme von zu reinigendem und zu desinfizierendem Spülgut, bei dem es sich beispielsweise um medizinische Instrumente und Geräte handeln kann. Der Spülraum ist über eine im Spülbottich ausgebildete Spülraumöffnung zugänglich, die mittels einer verschwenkbar am Spülbottich angeordneten Tür fluiddicht verschließbar ist.

Innerhalb des Spülraums ist eine Fluidabgabeeinrichtung angeordnet. Dabei dient die Fluidabgabeeinrichtung einerseits dazu, das zu reinigende Spülgut mit Spülflotte zum Zwecke der Reinigung und Desinfektion zu beaufschlagen. Nach erfolgter Reinigung und Desinfektion ist das Spülgut zu trocknen, zu welchem Zweck Trocknungsluft in den Spülraum eingeleitet wird. Die Abgabe der Trocknungsluft erfolgt ebenfalls über die Fluidabgabeeinrichtung. Damit dient die Fluidabgabeeinrichtung einerseits dazu, während des eigentlichen Reinigungs- und Desinfektionsvorganges Spülflotte auf das zu reinigende und zu desinfizierende Spülgut abzugeben sowie andererseits dazu, nach Abschluss eines solchen Reinigungs- und Desinfektionsvorganges das Spülgut mit Trocknungsluft zu beaufschlagen.
Ein solcher Reinigungsautomat ist beispielsweise in der JP 1126941 A beschrieben.

Um über die Fluidabgabeeinrichtung die Abgabe einerseits von Spülflotte und andererseits von Trocknungsluft zu gestatten, kommen bei Reinigungs- und Desinfektionsautomaten entsprechende Trenn- und Verteileinrichtungen zum Einsatz. Diese sorgen für eine Beschickung der Fluidabgabeeinrichtung entweder mit Spülflotte oder mit Trocknungsluft.

Aus dem Stand der Technik vorbekannte Trenn- und Verteileinrichtungen sind innerhalb des Spülraums ausgebildet und mit der zugehörigen Fluidabgabeeinrichtung vorzugsweise gekoppelt. Über entsprechende Speiseleitungen erfolgt eine Zuführung von Spülflotte bzw. Trocknungsluft an die Trenn- und Verteileinrichtung, die je nach wählbarer Stellung entweder Spülflotte oder Trocknungsluft über die Fluidabgabeeinrichtung zur Abgabe bringt.

Die vorbeschriebene Konstruktion hat sich bei Automaten kleinerer Bauart mit nur einer Fluidabgabeeinrichtung dem Grunde nach bewährt. Eine Übertragung dieser Konstruktion auf Automaten mit mehreren Fluidabgabeeinrichtungen ist allerdings nicht ohne Weiteres möglich und bringt im Übrigen erhebliche Nachteile mit sich. So ist die Kopplung von Fluidabgabeeinrichtung und Trenn- und Verteileinrichtung platzraubend. Da die Trenn- und Verteileinrichtung konstruktionsbedingt innerhalb des Spülraumes angeordnet ist, geht dadurch wertvoller Spülraum verloren. Mit steigender Anzahl der Fluidabgabeeinrichtungen verstärkt sich dieser Nachteil, da je Fluidabgabeeinrichtung eine Trenn- und Verteileinrichtung vorzusehen ist. Darüber hinaus vergrößert sich der Anschlussaufwand, da jede einzelne Trenn- und Verteileinrichtung an entsprechende Speiseleitungen anzuschließen ist. In der Konsequenz bedarf es einer Vielzahl einzelner Bauteile, was die Herstellungskosten steigen lässt, nicht zuletzt auch aufgrund der aufwendigeren Montage.

Aus dem Stand der Technik sind auch Konstruktionen bekannt geworden, die nicht über Trenn- und Verteileinrichtungen der vorbeschriebenen Art verfügen. Bei solchen Automaten handelt es sich typischerweise um Großgeräte, die über eine Mehrzahl von Fluidabgabeeinrichtungen verfügen, die typischerweise in Höhenrichtung übereinander angeordnet sind. Zur Versorgung dieser Fluidabgabeeinrichtungen entweder mit Spülflotte oder mit Trocknungsluft sind jeweils Speiseleitungen vorgesehen, die über außerhalb des Spülraums ausgebildete Verteileinrichtungen strömungstechnisch miteinander koppelbar bzw. voneinander entkoppelbar sind. Ein solcher Aufbau ist vergleichsweise kompliziert, störanfällig und so groß, dass er typischerweise nur bei Großgeräten Anwendung findet.
Im Übrigen hat sich bei dieser Konstruktion die zum Teil mangelhafte Hygienesicherheit als nachteilig herausgestellt. Um diese zu verbessern werden ist es bekannt, Ventileinrichtungen sowie Rückführleitungen in Form von Leckageleitungen eingesetzt, was den konstruktiven Aufbau zusätzlich verkompliziert und auch verteuert.
Ein Reinigungsautomat mit einer Mehrzahl von Fluidabgabeeinrichtungen, die in Höhenrichtung übereinander angeordnet sind, und denen über eine gemeinsame Speiseleitung entweder Spülflotte oder Trocknungsluft zugeführt werden kann, ist in der DE2556035 A1 beschrieben.

Ausgehend vom vorbeschriebenen Stand der Technik ist es die **Aufgabe** der Erfindung, einen Reinigungs- und Desinfektionsautomaten bereitzustellen, der unter Vermeidung der vorbeschriebenen Nachteile eine alternative Konstruktion zur Beschickung einer Fluidabgabeeinrichtung entweder mit Spülflotte oder mit Trocknungsluft bereitstellt.

Zur **Lösung** dieser Aufgabe wird mit der Erfindung vorgeschlagen ein Reinigungs- und Desinfektionsautomat, insbesondere für medizinische Instrumente und Geräte, gemäß Anspruch 1.

Der Reinigungs- und Desinfektionsautomat nach der Erfindung verfügt über eine Trenn- und Verteileinrichtung zur Beschickung einer innerhalb des Spülraums des Reinigungs- und Desinfektionsautomaten angeordneten Fluidabgabeeinrichtung. Diese Trenn- und Verteileinrichtung ist erfindungsgemäß außerhalb des Spülraums ausgebildet. In vorteilhafter Weise wird hierdurch eine optimierte Freihaltung des Spülraums erreicht, womit dieser im Betriebsfall ein Mehr an Spülgut aufnehmen kann.

Die nach der Erfindung vorgesehene Trenn- und Verteileinrichtung verfügt eingangsseitig über einen Spülflottenanschluss einerseits und über Trocknungsluftanschluss andererseits. Diese beiden Anschlüsse sind voneinander getrennt ausgebildet und an entsprechende Speiseleitungen angeschlossen. Im bestimmungsgemäßen Einsatzfall erfolgt eine Beschickung der Trenn- und Verteileinrichtung entweder über den Spülflottenanschluss mit Spülflotte oder über den Trocknungsluftanschluss mit Trocknungsluft.

Ausgangsseitig verfügt die Trenn- und Verteileinrichtung über wenigstens einen Ausgangsanschluss, der als gemeinsamer Ausgangsanschluss für Spülflotte einerseits und Trocknungsluft andererseits ausgebildet ist. An diesen Ausgangsanschluss ist die innerhalb des Spülraums des Reinigungs- und Desinfektionsautomaten angeordnete Fluidabgabeeinrichtung strömungstechnisch angekoppelt. Im bestimmungsgemäßen Verwendungsfall wird die Trenn- und Verteileinrichtung entweder mit Spülflotte oder mit Trocknungsluft eingangsseitig beaufschlagt. Die in die Trenn- und Verteileinrichtung eingeleitete Spülflotte bzw. die in die Trenn- und Verteileinrichtung eingeleitete Trocknungsluft durchströmt die Trenn- und Verteileinrichtung und wird ausgangsseitig über den Ausgangsanschluss abgegeben, von wo aus eine Beschickung der mit der Trenn- und Verteileinrichtung strömungstechnisch gekoppelten Fluidabgabeeinrichtung erfolgt, so dass eine Beaufschlagung des im Spülraum befindlichen Spülguts je nach Beschickung der Trenn- und Verteileinrichtung entweder mit Spülflotte oder mit Trocknungsluft erfolgt.

Die vorbeschriebene Konstruktion nach der Erfindung erweist sich neben der optimierten Spülraumnutzbarkeit auch insofern als vorteilhaft, als dass die Trenn- und Verteileinrichtung in einfacher Weise dazu ausgerüstet sein kann, an eine Mehrzahl von Fluidabgabeeinrichtungen angeschlossen zu werden. Je anzuschließender Fluidabgabeeinrichtung ist seitens der Trenn- und Verteileinrichtung ausgangsseitig ein jeweils gemeinsamer Ausgangsanschluss für Spülflotte einerseits und Trocknungsluft andererseits vorzusehen. In konstruktiv einfacher Weise ist so eine Beschickung auch mehrerer Fluidabgabeeinrichtungen möglich, und zwar ohne den konstruktiven Aufwand, wie er bei aus dem Stand der Technik bekannten Automaten bislang erforderlich ist.

Die erfindungsgemäße Konstruktion zeichnet sich insgesamt dadurch aus, dass sie kompakt und platzsparend ist, was eine optimierte Nutzung des Spülraums gestattet. Es kann ferner mit der erfindungsgemäßen Trenn- und Verteileinrichtung eine Mehrzahl von Ausgangsanschlüssen vorgesehen sein, was den Anschluss einer Mehrzahl von Fluidabgabeeinrichtungen gestattet. Die erfindungsgemäße Konstruktion kann daher auch in Kombination mit Großgeräten Verwendung finden, insbesondere solchen, die über eine Mehrzahl von in Lagen übereinander angeordnete Fluidabgabeeinrichtungen verfügen. Dabei ist die nach der Erfindung vorgesehene Trenn- und Verteileinrichtung in ihrer Verwendung hygienisch unbedenklich. Auf gesonderte Ventileinrichtungen, Bypassleitungen, Leckageleitungen und/oder dergleichen kann im Unterschied zum Stand der Technik verzichtet werden, womit das Risiko hinsichtlich hygienisch bedenklicher Verunreinigungen minimiert ist.

Die Trenn- und Verteileinrichtung nach der Erfindung verfügt über zwei Kammern. Die eine Kammer ist mit dem Spülflottenanschluss und die zweite Kammer mit dem Trocknungsluftanschluss ausgerüstet. Die beiden Kammern sind mittels einer schwenkbar ausgebildeten ersten Klappe strömungstechnisch koppelbar bzw. entkoppelbar. Eine der beiden Kammern, vorzugsweise die ersten Kammer, ist ausgangsseitig mit dem Ausgangsanschluss ausgerüstet, so dass in Abhängigkeit der Klappenstellung entweder der Spülflottenanschluss oder der Trocknungsluftanschluss mit dem wenigstens einen Ausgangsanschluss gekoppelt ist.

Die erste Klappe ist bevorzugter Weise selbsttätig klappend ausgebildet, d. h. ohne weiteren Antrieb ausgestaltet. Bei einer Beschickung der Trenn- und Verteileinrichtung mit Spülflotte wird die erste Klappe in Folge der einströmenden Spülflotte in eine für die Spülflotte geöffnete Stellung verschwenkt. Die Spülflotte kann die erste Kammer, das heißt die Spülflottenkammer der Trenn- und Verteileinrichtung durchströmen, wodurch sie zum Ausgangsanschluss geleitet wird. Ein Eindringen von Spülflotte in die zweite Kammer, das heißt die Trocknungsluftkammer ist bis auf eine gewollte Spülflottenabzweigung unterbunden.

Bei einer Beschickung der Trenn- und Verteileinrichtung mit Trocknungsluft befindet sich die erste Klappe in ihrer für die Trocknungsluft offenen Stellung. Diese Stellung nimmt die erste Klappe vorzugsweise von selbst nach Abschluss eines Spülvorgangs ein, d.h. sobald keine Einströmung von Spülflotte mehr erfolgt. Alternativ ist es aber auch denkbar, dass die erste Klappe durch die einströmende Trocknungsluft bewegt wird und dadurch diese Stellung einnimmt. Mittels Klappe sind die erste Kammer und die zweite Kammer strömungstechnisch miteinander gekoppelt, so dass die in die zweite Kammer eingebrachte Trocknungsluft aus der zweiten Kammer in die erste Kammer überströmen und alsdann über den Ausgangsanschluss abgegeben werden kann.

Der Vorteil der vorbeschriebenen Klappen-Lösung ist insbesondere die sich dadurch ergebende strömungsoptimierte Auslegung der gesamten Trenn- und Verteileinrichtung. Insbesondere der Strömungsweg für die Spülflotte ist im Wesentlichen gradlinig verlaufend ausgebildet.

Gemäß einem weiteren Merkmal der Erfindung verfügt die Trenn- und Verteileinrichtung über eine zweite Klappe. Diese zweite Klappe ist anschlussseitig der Trocknungsluft ausgebildet. Sie verschließt den Trocknungsluftanschluss und stellt in ihrer Verschlussstellung sicher, dass in die Trenn- und Verteileinrichtung einströmende Spülflotte nicht ungewollt über den Trocknungsluftanschluss aus der Trenn- und Verteileinrichtung austreten kann.

Die zweite Klappe ist federvorgespannt in Verschlussstellung gehalten. Bei geöffneter erster Klappe, wenn also keine Trocknungsluft, sondern Spülflotte in die Trenn- und Verteileinrichtung einströmt, ist die zweite Klappe verschlossen, womit in schon vorbeschriebener Weise sichergestellt ist, dass nicht ungewollt Spülflotte durch den Trocknungsluftanschluss die Trenn- und Verteileinrichtung verlassen kann. Sobald die Spülflottenzufuhr beendet ist, kann die Trenn- und Verteileinrichtung mit Trocknungsluft beaufschlagt werden. Dabei drückt die einströmende Trocknungsluft entgegen der Federvorspannung auf die zweite Klappe ein, was zu einer Verschwenkung derselben führt, so dass Trocknungsluft in die Trenn- und Verteileinrichtung einströmen kann. Die erste Klappe ist nach Beendigung der Spülflottenzufuhr bereits in die für Spülflotte geschlossenen Stellung zurückgeschwenkt oder wird in Folge der einströmenden Trocknungsluft in diese Stellung beiseite geschwenkt, was in jedem Fall zu einer strömungstechnischen Verbindung der beiden Kammern der Trenn- und Verteileinrichtung mit der Folge führt, dass die eingeströmte Trocknungsluft über den Ausgangsanschluss an die daran angeschlossene Fluidabgabeeinrichtung geleitet wird.

Die Trenn- und Verteileinrichtung verfügt gemäß einer besonders bevorzugten Ausführungsform über einen weiteren Ausgangsanschluss. Dieser weitere Ausgangsanschluss ist vorzugsweise im Bereich der zweiten Kammer angeordnet und mündet direkt innerhalb des Spülraumes, die zweite Kammer somit unmittelbar mit dem Spülraum verbindend. Der weitere Ausgangsanschluss ist also nicht an eine separate Fluidabgabeeinrichtung angeschlossen.
Über diesen weiteren Ausgangsanschluss kann insbesondere eine Beschickung des Spülraums mit Trocknungsluft zum Zwecke der Außentrocknung erfolgen. "Außentrocknung" meint dabei, dass von den Fluidabgabeeinrichtungen ansonsten nicht beaufschlagbare Bereiche des Spülbottichs und/oder des im Spülraum befindlichen Spülguts mit Trocknungsluft beaufschlagt werden können. Typischerweise ist eine solche Außentrocknung bei Gefäßen wie zum Beispiel Glaskolben erforderlich, da diese zum Zwecke der Innenraumreinigung einer Fluidabgabeeinrichtung direkt zugeordnet sind. Bei einer Beaufschlagung dieser Fluidabgabeeinrichtung mit Trocknungsluft wird zwar eine Innenraumtrocknung, nicht aber eine Außentrocknung erreicht. Für eine solche Außentrocknung dient der weitere Ausgangsanschluss in der schon vorbeschriebenen Weise.

Um zu verhindern, dass über den weiteren Ausgangsanschluss ein zu großer Volumenstrom an Trocknungsluft in den Spülraum abgeführt wird, ist gemäß einem weiteren Merkmal der Erfindung vorgesehen, dass der weitere Ausgangsanschluss bei geöffneter zweiter Klappe zumindest teilweise durch die zweite Klappe geschlossen ist. Auf diese Weise ist sichergestellt, dass die Trocknungsluft in einem vorgebbaren Aufteilungsverhältnis zum einen der angeschlossenen Fluidabgabeeinrichtung sowie zum anderen dem weiteren Ausgangsanschluss zugeführt wird.

Eine wesentliche Funktion des weiteren Ausgangsanschlusses ist es darüber hinaus, dass er eine direkte Anbindung der zweiten Kammer an den Spülraum schafft und dabei vorzugsweise, insbesondere durch seine Anordnung und Größe, eine permanente Umspülung der Trenn- und Verteileinrichtung im Spülbetrieb ermöglicht, insbesondere unmittelbar unterstützt durch die Spülmechanik im unmittelbar angrenzenden Spülrauminnenraum. Damit werden eine Keimbildung in der Trenn- und Verteileinrichtung, insbesondere in der zweiten Kammer, und eine etwaige Rekontamination des Spülguts bzw. der Spülflotte wirksam verhindert.

Insgesamt wird durch die Erfindung damit eine überraschend einfache und gleichzeitig in Bezug auf die Hygieneanforderungen besonders sichere und trotzdem platzsparende Konstruktion eines Reinigungs- und Desinfektionsautomaten angegeben.

Die Trenn- und Verteileinrichtung ist bevorzugter Weise als eigenständig handhabbare Baukomponente ausgebildet. Dies vereinfacht insbesondere eine Montage, da eine Vormontage der Trenn- und Verteileinrichtung gestattet ist.

Weitere Merkmale und Vorteile der Erfindung ergeben sich aus der nachfolgenden Beschreibung anhand der Figuren. Dabei zeigen:
- Figur 1: in schematischer Ansicht einen Reinigungs- und Desinfektionsautomaten nach der Erfindung;
- Figur 2: in schematischer Darstellung eine Trenn- und Verteileinrichtung nach der Erfindung;
- Figur 3: in perspektivischer Ansicht eine Trenn- und Verteileinrichtung nach der Erfindung und
- Figur 4: in einer Ausschnittsdarstellung die Trenn- und Verteileinrichtung nach Figur 3.

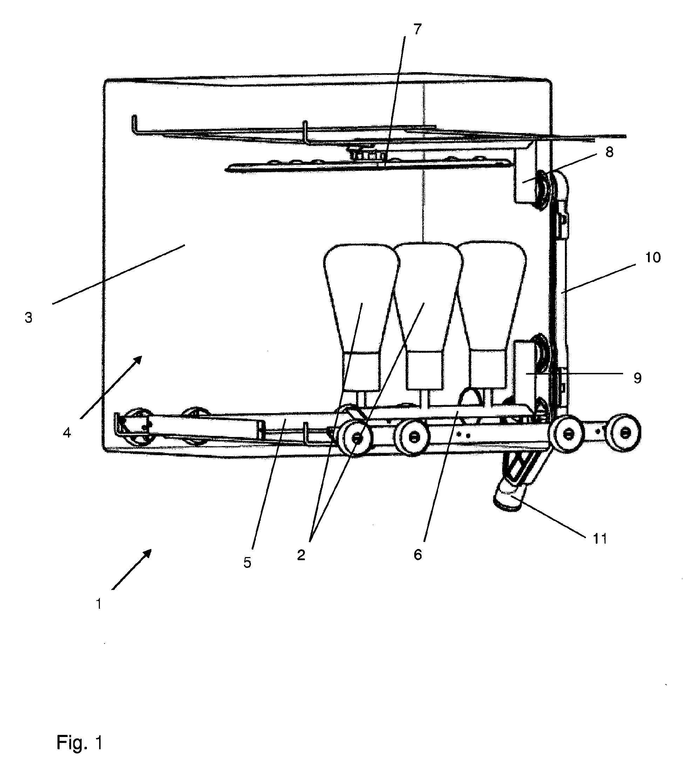

Figur 1 lässt in rein schematischer Darstellung einen Reinigungs- und Desinfektionsautomaten 1 nach der Erfindung erkennen. Dieser verfügt über einen Spülbottich 3, der einen Spülraum 4 bereitstellt. Der Spülraum 4 ist mittels einer in den Figuren nicht näher dargestellten Tür fluiddicht verschließbar.

Im bestimmungsgemäßen Verwendungsfall dient der Spülraum 4 der Aufnahme von zu reinigendem Spülgut, bei dem es sich beispielsweise um medizinische Instrumente und Geräte 2 handeln kann. Im gezeigten Ausführungsbeispiel sind als zu reinigendes medizinisches Gerät 2 Glaskolben dargestellt.

Um eine vereinfachte Beschickung des Spülraums 4 mit zu reinigendem Spülgut zu erreichen, kommen bevorzugter Weise aus dem Spülraum 4 herausfahrbare Wagen 5 zum Einsatz, die das zu reinigende Spülgut tragen.

Zum Zwecke der Beschickung des Spülraums 4 mit Spülflotte oder mit Trocknungsluft kommt eine Fluidabgabeeinrichtung zum Einsatz, die innerhalb eines Spülraumes 4 ausgebildet ist. Der Reinigungs- und Desinfektionsautomat 1 gemäß der Ausführungsform nach Figur 1 verfügt über zwei solche Fluidabgabeeinrichtungen, nämlich eine erste Fluidabgabeeinrichtung 6 und eine zweite Fluidabgabeeinrichtung 7.

Die erste Fluidabgabeeinrichtung 6 ist mit dem Wagen 5 gekoppelt. Sie kann zusammen mit diesem aus dem Spülraum 4 zwecks Bestückung mit Spülgut heraus verfahren werden. Dabei ist die erste Fluidabgabeeinrichtung 6 im gezeigten Ausführungsbeispiel derart ausgebildet, dass eine Beschickung der einzelnen Glaskolben mit Spülflotte oder Trocknungsluft zur Innenraumreinigung erfolgen kann. Die erste Fluidabgabeeinrichtung 6 verfügt deshalb über einzelne Fluidabgabestellen, wobei jeder Fluidabgabestelle im bestimmungsgemäßen Verwendungsfall einer der im Ausführungsbeispiel nach Figur 1 gezeigten Glaskolben zugeordnet ist.

Die zweite Fluidabgabeeinrichtung 7 ist als drehbarer Sprüharm ausgebildet. Dieser dient der Beschickung der Glaskolben 2 mit Spülflotte oder Trocknungsluft zum Zwecke der Reinigung von außen.

Die beiden Fluidabgabeeinrichtungen 6 und 7 sind an eine gemeinsame Trenn- und Verteileinrichtung 10 angeschlossen, die erfindungsgemäß außerhalb des Spülarms 4 ausgebildet ist.

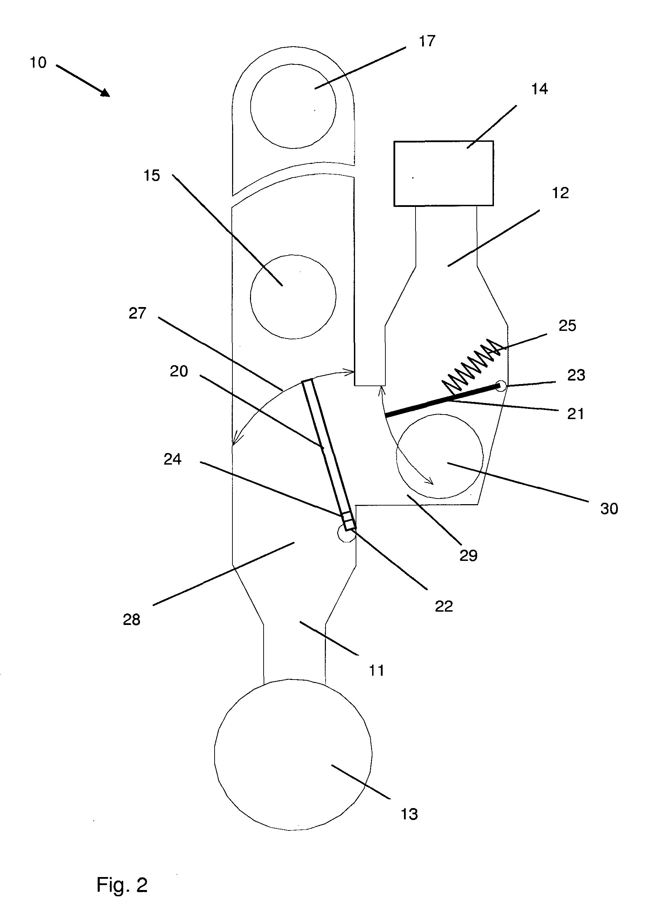

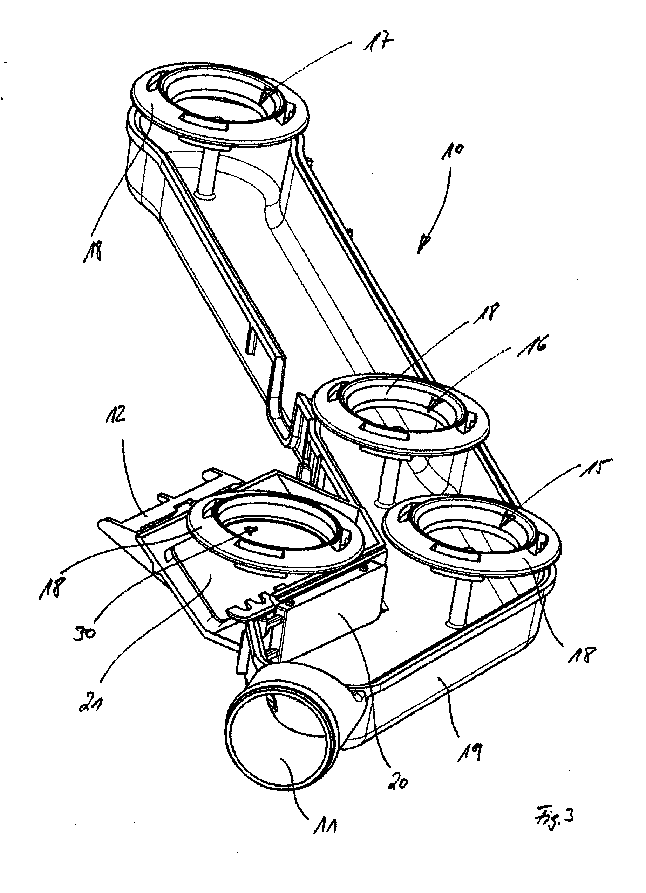

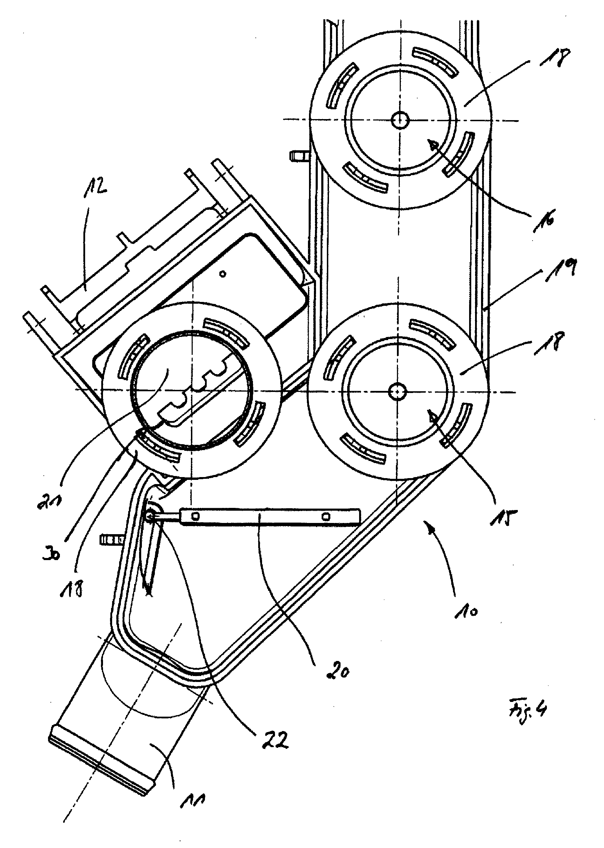

Die Trenn- und Verteileinrichtung 10 ist in den Figuren 2, 3 und 4 im Detail dargestellt, wobei Figur 2 eine rein schematische Darstellung betrifft.

Die Trenn- und Verteileinrichtung 10 verfügt über ein Gehäuseteil 19. Dieses Gehäuseteil 19 stellt zwei Kammern, nämlich eine erste Kammer 28 und eine zweite Kammer 29 bereit. Die erste Kammer 28 ist als Spülflottenkammer und die zweite Kammer 29 als Trocknungsluftkammer zu bezeichnen. Die beiden Kammern 28 und 29 sind mittels einer um eine Schwenkachse 22 verschwenkbaren Schwenkklappe 20 strömungstechnisch koppelbeziehungsweise entkoppelbar. Die Schwenkklappe 20 kann in Richtung des Doppelpfeils 27 hin- und her verschwenken, wie sich insbesondere aus der Darstellung nach Figur 2 ergibt.

Das Gehäuseteil 19 stellt eingangsseitig einen Spülflottenanschluss 11 bereit. Dieser ist strömungstechnisch an eine Umwälzpumpe 13 angeschlossen, über die Spülflotte im bestimmungsgemäßen Verwendungsfall gefördert wird.

Das Gehäuseteil 19 weist eingangsseitig des Weiteren einen Trocknungsanschluss 12 auf. Dieser ist strömungstechnisch an ein Trocknungsluftaggregat 14 gekoppelt, über welches im Betriebsfall Trocknungsluft in die Trenn- und Verteilerdichtung 10 eingespeist wird.

Die beiden Eingangsanschlüsse, das heißt der Spülflottenanschluss 11 und der Trocknungsluftanschluss 12 sind voneinander getrennt ausgebildet.

Ausgangsseitig verfügt die Trenn- und Verteileinrichtung 10 über Ausgangsanschlüsse, wobei im gezeigten Ausführungsbeispiel drei Ausgangsanschlüsse 15, 16 und 17 dargestellt sind. Dabei dienen die Ausgangsanschlüsse 15 oder 16 der Kopplung der Trenn- und Verteileinrichtung 10 mit der ersten Fluidabgabeeinrichtung 6, zu welchem Zweck diese Fluidabgabeeinrichtung über eine entsprechend ausgebildete Anschlussleitung 9 verfügt, wie sich insbesondere aus der Darstellung nach Figur 1 ergibt.

Der Ausgangsanschluss 17 dient indes der strömungstechnischen Verbindung der Trenn- und Verteileinrichtung 10 mit der zweiten Fluidabgabeeinrichtung 7, zu welchem Zweck diese über eine entsprechende Anschlussleitung 8 verfügt, welcher Sachzusammenhang sich ebenfalls insbesondere aus der Darstellung nach Figur 1 ergibt.

Die Trenn- und Verteileinrichtung 10 verfügt über eine weitere Schwenkklappe 21. Diese ist innerhalb der zweiten Kammer 29 der Trenn- und Verteileinrichtung 10, das heißt der Trocknungsluftkammer angeordnet. Sie ist um die Schwenkachse 23 verschwenkbar, und zwar in Richtung des Doppelpfeils 26. Die Schwenkklappe 21 steht unter Federvorspannung, zu welchem Zweck die als Zugfeder ausgebildete Feder 25 vorgesehen ist. In ihrer Verschlussstellung dichtet die Schwenkklappe 21 den Trocknungsanschluss 12 ab, so dass in der Trenn- und Verteileinrichtung 10 befindliche Spülflotte nicht ungewollt über den Trocknungsluftanschluss 12 nach außen gelangen kann.

Die Trenn- und Verteileinrichtung 10 verfügt über eine weitere Ausgangsöffnung 30. Diese ist innerhalb der zweiten Kammer 29 ausgebildet. Über diese weitere Ausgangsöffnung 30 kann eine Beschickung des Spülraums 4 mit Spülflotte oder Trocknungsluft erfolgen, und zwar direkt, das heißt ohne Zwischenschaltung einer Fluidabgabeeinrichtung. Über diese weitere Ausgangsöffnung 30 erfolgt insbesondere eine Abgabe von Trocknungsluft zum Zwecke einer Außentrocknung sowie eine Umspülung der Trenn- und Verteilereinrichtung im Spülbetrieb. im Steht auf Seite Dabei ist bevorzugter Weise die zweite Schwenkklappe 21 derart ausgebildet, dass diese in geöffneter Stellung zumindest teilweise die weitere Ausgangsöffnung 30 abdeckt, so dass im Falle einer Beschickung der Trenn- und Verteileinrichtung 10 mit Trocknungsluft nicht sämtliche Trocknungsluft über die weitere Ausgangsöffnung 30 zur Außentrocknung abgegeben wird, sondern auch ein Teil der Trocknungsluft zu den Ausgangsanschlüssen 15 und 16 gelangt, so dass über die daran angeschlossene Fluidabgabeeinrichtung 6 eine Innentrocknung des im vorliegenden Fall beispielhaft als Glaskolben dargestellten medizinischen Geräts 2 stattfinden kann.

Die Schwenkklappe 20 ist selbstklappend, das heißt ohne jeglichen Antrieb ausgebildet. Die Schwenkklappe 20 verschwenkt je nach Beschickung der Trenn- und Verteileinrichtung 10 mit Spülflotte über den Spülflottenanschluss 11 beziehungsweise mit Trocknungsluft über den Trocknungsanschluss 12. Bei einer Beschickung der Trenn- und Verteileinrichtung 10 mit Spülflotte 11 verschwenkt die Schwenkklappe 20 mit Bezug auf die Zeichnungsebene nach Figur 2 nach rechts, so dass die Spülflotte nach Eintritt in die Trenn- und Verteileinrichtung 10 über den Spülflottenanschluss 11 direkt zu den Ausgangsanschlüssen 15, 16 und 17 strömen kann. Dabei sorgt die Stellung der Klappe 20 dafür, dass ein optimierter Strömungsweg für die Spülflotte in Richtung der Ausgangsanschlüsse 15, 16 und 17 bereitgestellt ist. Ferner ist in dieser Stellung der Schwenkklappe dafür Sorge getragen, dass Spülflotte im Wesentlichen nicht in die vom Gehäuseteil 19 bereitgestellte zweite Kammer 29 einströmt. Dabei schließt die Schwenkklappe 20 die zweite Kammer 29 bewusst nicht fluiddicht ab, damit der Teilbereich der zweiten Klammer 29 zwischen Schwenkklappe 20 und Schwenkklappe 21 regelmäßig von Spülflotte mit durchströmt wird, wodurch in vorteilhafter Weise eine regelmäßige Reinigung und Desinfektion nicht nur der ersten Kammer 28, sondern auch der zweiten Kammer 29 sichergestellt ist und außerdem ein optimales Leerlaufverhalten gewährleistet ist. In die zweite Kammer 29 zum Zwecke der Reinigung und Desinfektion einströmende Spülflotte kann über die Ablauföffnung 24 in der Schwenkklappe 20 und/oder die weitere Ausgangsöffnung 30 zurückströmen. Über die weitere Ausgangsöffnung 30 kann im bestimmungsgemäßen Verwendungsfall des Reinigungs- und Desinfektionsautomaten 1 Spülflotte auch aus dem Spülraum 4 in die Trenn- und Verteileinrichtung 10 gelangen und somit deren Umspülung gewährleisten.

Damit in die zweite Kammer 29 einströmende Spülflotte nicht ungewollt über den Trocknungsluftanschluss 12 abströmen kann, ist die zweite Schwenkklappe 21 vorgesehen. Diese dient einem fluiddichten Verschluss des Trocknungsluftanschlusses 12. Dabei ist die Schwenkklappe 21 mittels einer Zugfeder 25 federvorgespannt und befindet sich in ihrer Normalstellung in Verschlussstellung.

Im bestimmungsgemäßen Fall der Trocknung wird mittels des Trocknungsaggregats 14 Trocknungsluft erzeugt und in die Trenn- und Verteileinrichtung 10 eingebracht. Aufgrund der Einströmung von Trocknungsluft wird die Schwenkklappe 21 entgegen ihrer Federvorspannung verschwenkt, was das Eindringen der Trocknungsluft gestattet. Die erste Schwenkklappe 20 wird gemäß einem ersten Ausführungsbeispiel nach Abschluss eines Spülvorgangs, sobald keine weitere Spülflotte einströmt, durch Schwerkraft in eine für Trocknungsluft geöffneten Stellung geschwenkt oder wird gemäß einem zweiten Ausführungsbeispiel in Folge der Beschickung mit Trocknungsluft verschwenkt, und zwar mit Bezug auf die Zeichnungsebene nach Figur 2 nach links. In Folge dieser Verschwenkung kann die Trocknungsluft über die zweite Kammer 29 in die erste Kammer 28 zu den Ausgangsanschlüssen 15, 16 und 17 gelangen. Von hier aus wird die Trocknungsluft sodann an die Fluidabgabeeinrichtungen 6 und 7 weitergeleitet. Ein Teil der Trocknungsluft gelangt auch über die weitere Ausgangsöffnung 30 in den Spülraum 4. Dabei ist die Schwenkklappe 21 derart ausgestaltet, dass im Falle ihrer Verschwenkung ein Teil der weiteren Ausgangsöffnung 30 verschlossen ist, so dass nicht die gesamte Trocknungsluft über die weitere Ausgangsöffnung 30 in den Spülraum 4 abgegeben wird.

Zur Anbindung der Trenn- und Verteileinrichtung 10 am Spülbottich 3 dienen insbesondere mit den jeweiligen Ausgangsanschlüssen korrespondierend zusammenwirkende Flanschringe 18. Im montierten Zustand, wie er schematisch in Figur 1 dargestellt ist, befindet sich das Gehäuseteil 19 der Trenn- und Verteileinrichtung 10 außerhalb des Spülraums 4, wobei spülrauminnenseitig die den einzelnen Ausgangsöffnungen der Trenn- und Verteileinrichtung 10 zugeordneten Flanschringe 18 angeordnet sind. Im montierten Zustand befindet sich also die Bottichraumwand zwischen dem Gehäuseteil 19 und den Flanschringen 18 der Trenn- und Verteileinrichtung 10.

### Bezugszeichenliste

- 1: Reinigungs- und Desinfektionsautomat
- 2: medizinisches Gerät
- 3: Spülbottich
- 4: Spülraum
- 5: Wagen
- 6: erste Fluidabgabeeinrichtung
- 7: zweite Fluidabgabeeinrichtung
- 8: Anschlussleitung
- 9: Anschlussleitung
- 10: Trenn- und Verteileinrichtung
- 11: Spülflottenanschluss
- 12: Trocknungsluftanschluss
- 13: Umwälzpumpe
- 14: Trocknungsluftaggregat
- 15: Ausgangsanschluss
- 16: Ausgangsanschluss
- 17: Ausgangsanschluss
- 18: Flanschring
- 19: Gehäuseteil
- 20: Schwenkklappe
- 21: Schwenkklappe
- 22: Schwenkachse
- 23: Schwenkachse
- 24: Ablauföffnung
- 25: Feder
- 26: Doppelpfeil
- 27: Doppelpfeil
- 28: erste Kammer
- 29: zweite Kammer
- 30: weitere Ausgangsöffnung

## Patentansprüche

1. Reinigungs- und Desinfektionsautomat insbesondere für medizinische Instrumente und Geräte, mit einem Spülbottich (3), der einen mit einer Tür fluiddicht verschließbaren Spülraum (4) bereitstellt, mit einer im Spülraum (4) angeordneten Fluidabgabeeinrichtung (6, 7), die der Abgabe von Spülflotte einerseits und von Trocknungsluft andererseits dient, sowie mit einer außerhalb des Spülraums (4) ausgebildeten Trenn- und Verteileinrichtung (10) zur Beschickung der Fluidabgabeeinrichtung (6, 7) entweder mit Spülflotte oder mit Trocknungsluft, wobei die Trenn- und Verteileinrichtung (10) eingangsseitig einen Spülflottenanschluss (11) und einen davon getrennt ausgebildeten Trocknungsluftanschluss (12) sowie ausgangsseitig einen gemeinsamen Ausgangsanschluss (15, 16, 17) für Spülflotte einerseits und Trocknungsluft andererseits aufweist
**dadurch gekennzeichnet,**
**dass** die Trenn- und Verteileinrichtung (10) einen weiteren Ausgangsanschluss (30) aufweist zur Beschickung des Spülraums (4) mit Trocknungsluft zum Zwecke der Außentrocknung des Spülgutes, wobei der weitere Ausgangsanschluss (30) so ausgebildet ist, dass im Spülbetrieb Spülflotte durch ihn in die Trenn- und Verteileinrichtung (10) gelangt.

2. Automat nach Anspruch 1, **dadurch gekennzeichnet, dass** eine Mehrzahl von gemeinsamen Ausgangsanschlüssen (15, 16, 17) für Spülflotte einerseits und Trocknungsluft andererseits vorgesehen ist, wobei jedem Ausgangsanschluss (15, 16, 17) eine Fluidabgabeeinrichtung (6, 7) zugeordnet ist.

3. Automat nach Anspruch 1 oder 2, **gekennzeichnet durch** eine Klappe (20), die in Abhängigkeit ihrer Stellung den Spülflottenanschluss (11) oder den Trocknungsluftanschluss (12) mit dem Ausgangsanschluss (15, 16, 17) koppelt.

4. Automat nach Anspruch 3, **dadurch gekennzeichnet, dass** die Klappe (20) selbsttätig klappend ausgebildet ist.

5. Automat nach einem der vorhergehenden Ansprüche, **gekennzeichnet durch** eine zweite Klappe (21), die anschlussseitig der Trocknungsluft ausgebildet ist.

6. Automat nach Anspruch 5, **dadurch gekennzeichnet, dass** die zweite Klappe (21) federvorgespannt in Verschlussstellung gehalten ist.

7. Automat nach Anspruch 5 oder 6, **dadurch gekennzeichnet, dass** die zweite Klappe (21) bei geöffneter erster Klappe (20) in Verschlussstellung gehalten ist.

8. Automat nach einem der vorhergehenden Ansprüche, **dadurch gekennzeichnet, dass** der weitere Ausgangsanschluss (30) bei geöffneter zweiter Klappe (21) zumindest teilweise durch die zweite Klappe (21) geschlossen ist.

9. Automat nach einem der vorhergehenden Ansprüche, **dadurch gekennzeichnet, dass** die Trenn- und Verteileinrichtung (10) als eigenständig handhabbare Baugruppe ausgebildet ist.

## Claims

1. Automatic cleaning and disinfection machine, in particular for medical instruments and appliances, comprising a rinsing tub (3), which provides a rinsing chamber (4) which can be closed in a fluid-tight manner using a door, a fluid discharge device (6, 7) arranged in the rinsing chamber (4) and serving to discharge both rinsing solution and drying air, and a separation and distribution device (10), formed outside the rinsing chamber (4), for loading the fluid discharge device (6, 7) either with rinsing solution or with drying air, the separation and distribution device (10) comprising at the inlet side a rinsing solution supply (11) and a drying air supply (12), which is separated therefrom, and comprising at the outlet side a common outlet connection (15, 16, 17) for both rinsing solution and drying air,
**characterised in that**
the separation and distribution device (10) comprises a further outlet connection (30) for loading the rinsing chamber (4) with drying air in order to externally dry the item to be rinsed, the further outlet connection (30) being formed such that, during the rinsing operation, rinsing solution reaches the separation and distribution device (10) through said outlet connection.

2. Automatic machine according to claim 1, **characterised in that** a plurality of common outlet connections (15, 16, 17) is provided for both the rinsing solution and the drying air, a fluid discharge device (6, 7) being associated with each outlet connection (15, 16, 17).

3. Automatic machine according to either claim 1 or claim 2, **characterised by** a flap (20) which, depending on its position, couples either the rinsing fluid supply (11) or the drying air supply (12) to the outlet connection (15, 16, 17).

4. Automatic machine according to claim 3, **characterised in that** the flap (20) is formed so as to flap automatically.

5. Automatic machine according to any of the preceding claims, **characterised by** a second flap (21), which is formed at the outlet side of the drying air.

6. Automatic machine according to claim 5, **characterised in that** the second flap (21) is held in the closed position by being spring pre-tensioned.

7. Automatic machine according to either claim 5 or claim 6, **characterised in that** the second flap (21) is held in the closed position when the first flap (20) is open.

8. Automatic machine according to any of the preceding claims, **characterised in that** the further outlet connection (30) is closed at least in part by the second flap (21) when the second flap (21) is open.

9. Automatic machine according to any of the preceding claims, **characterised in that** the separation and distribution device (10) is formed as an assembly that can be handled independently.

## Revendications

1. Appareil automatique de nettoyage et de désinfection, en particulier pour instruments et appareils médicaux, avec un bac de rinçage (3) qui réalise un espace de rinçage (4) pouvant être fermé de façon étanche aux fluides par une porte, avec un dispositif de délivrance de fluide (6, 7), disposé dans l'espace de rinçage (4), qui sert à la délivrance d'eau de rinçage d'une part et d'air de séchage d'autre part, ainsi qu'avec un dispositif de séparation et de distribution (10), constitué à l'extérieur de l'espace de rinçage (4), pour l'alimentation du dispositif de délivrance de fluide (6, 7) soit avec de l'eau de rinçage, soit avec de l'air de séchage, le dispositif de séparation et de distribution (10) présentant, côté entrée, un raccordement d'eau de rinçage (11) et un raccordement d'air de séchage (12) constitué séparément du raccordement précédent ainsi que, côté sortie, un raccordement de sortie (15, 16, 17) commun pour l'eau de rinçage d'une part et l'air de séchage d'autre part,
**caractérisé en ce que**
le dispositif de séparation et de distribution (10) présente un autre raccordement de sortie (30) pour l'alimentation de l'espace de rinçage (4) avec de l'air de séchage en vue du séchage extérieur de l'objet à rincer, l'autre raccordement de sortie (30) étant constitué de telle sorte que, dans le mode rinçage, de l'eau de rinçage parvient à travers lui dans le dispositif de séparation et de distribution (10).

2. Appareil automatique selon la revendication 1, **caractérisé en ce qu'**une pluralité de raccordements de sortie (15, 16, 17) communs est prévue pour de l'eau de rinçage d'une part et de l'air de séchage d'autre part, un dispositif de délivrance de fluide (6, 7) étant affecté à chaque raccordement de sortie (15, 16, 17).

3. Appareil automatique selon la revendication 1 ou 2, **caractérisé par** un clapet (20) qui, en fonction de sa position, couple le raccordement d'eau de rinçage (11) ou le raccordement d'air de séchage (12) au raccordement de sortie (15, 16, 17).

4. Appareil automatique selon la revendication 3, **caractérisé en ce que** le clapet (20) est constitué de façon à manoeuvrer automatiquement.

5. Appareil automatique selon une des revendications précédentes, **caractérisé par** un deuxième clapet (21), qui est constitué sur le côté raccordement de l'air de séchage.

6. Appareil automatique selon la revendication 5, **caractérisé en ce que** le deuxième clapet (21) est retenu dans la position de fermeture par prétension par ressort.

7. Appareil automatique selon la revendication 5 ou 6, **caractérisé en ce que** le deuxième clapet (21) est retenu dans la position de fermeture lorsque le premier clapet (20) est ouvert.

8. Appareil automatique selon une des revendications précédentes, **caractérisé en ce que**, lorsque le deuxième clapet (21) est ouvert, l'autre raccordement de sortie (30) est fermé au moins partiellement par le deuxième clapet (21).

9. Appareil automatique selon une des revendications précédentes, **caractérisé en ce que** le dispositif de séparation et de distribution (10) est constitué en tant que module pouvant être mis en oeuvre séparément.
